(19) Europäisches Patentamt
European Patent Office
Office européen des brevets

(11) **EP 4 711 497 A1**

(12) **EUROPEAN PATENT APPLICATION**

(43) Date of publication:
**18.03.2026 Bulletin 2026/12**

(21) Application number: **25199524.7**

(22) Date of filing: **02.09.2025**

(51) International Patent Classification (IPC):
**C25B 1/04** (2021.01)  **C25B 9/23** (2021.01)
**C25B 15/027** (2021.01)  **C25B 15/029** (2021.01)
**G01N 33/00** (2006.01)

(52) Cooperative Patent Classification (CPC):
**C25B 1/04; B01D 53/8671; C25B 9/00;
C25B 15/023; C25B 15/029; C25B 15/083;
G01N 33/005;** C25B 9/23

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **03.09.2024 IT 202400019663**

(71) Applicant: **MicroPROGEL S.R.L.**
**35038 Torreglia (PD) (IT)**

(72) Inventor: **Gaffo, Valter**
**35038 Torreglia (PD) (IT)**

(74) Representative: **Citron, Massimiliano**
**Via Primo Maggio, 6**
**31020 San Fior (TV) (IT)**

(54) **METHOD AND APPARATUS FOR DETECTING THE PRESENCE OF HYDROGEN IN THE OXYGEN STREAM GENERATED BY A PEM CELL**

(57)    A method is described for detecting the presence of hydrogen in the oxygen stream generated by a PEM cell, wherein the PEM cell comprises a membrane permeable to $H^+$ ions , a first inlet conduit for water, a second outlet conduit for hydrogen, and a third outlet conduit for the generated oxygen. The hydrogen and the oxygen being produced by the molecular dissociation of water inside the PEM cell.

In the method the temperature of a catalyst placed in contact with said oxygen stream, is detected.

Fig. 1

EP 4 711 497 A1

**Description**

The invention relates to a method and apparatus for detecting the presence of hydrogen in the oxygen stream generated by a PEM cell, in particular for the early detection of potentially dangerous malfunctions in the PEM cell.

PEM cell-based hydrogen generators typically use polymer membranes and operate according to the following electrochemical principle:

1. Water is introduced into the PEM cell;
2. Inside the cell, water undergoes a process of electrolysis, separating into pure hydrogen ($H_2$) and oxygen ($O_2$);
3. This separation occurs through an internal polymeric membrane, permeable only to $H^+$ ions (protons);
4. The overall chemical reaction can be represented as:

$$2H_2O \rightarrow 2H_2 + O_2;$$

5. The polymer membrane acts as a solid electrolyte, facilitating the transport of protons and effectively separating the hydrogen from the oxygen thus produced.

This electrochemical process allows the production of high-purity hydrogen, making PEM cells particularly suitable for applications requiring high-quality hydrogen.

The oxygen produced leaves the PEM cell with the output water. Oxygen is typically considered a waste product, while hydrogen passes through the membrane and leaves the PEM cell in gaseous form in a separate conduit. The PEM cell may also have multiple membrane layers internally.

Fatigue or aging can cause a hole in the membrane (or one of the membranes) due, for example, to the pressure it is subjected to by the generated hydrogen. In this case, the generated hydrogen ends up in the oxygen stream and leaves the PEM cell with it, compromising the efficiency of the latter. Over time, the oxygen and hydrogen tend to recombine at the hole, often also with micro-explosions, enlarging the hole, which gets physically burned. Not only does the PEM cell quickly cease to function, but especially in high-power (kW) generators, considerable quantities of hydrogen could be released along with oxygen, creating potentially dangerous mixtures.

The problem is therefore to detect whether there is hydrogen in the stream of generated oxygen exiting the PEM cell, as described for example in US8669017. For this purpose, it is common practice to use a hydrogen sensor. However, the humid and saturated environment of this line creates unfavorable conditions for the sensor's operation. To overcome this problem, two alternatives are generally used, both with significant disadvantages:

1. the use of water-resistant sensors, which however entails considerable system complexity and additional costs, or
2. drying out the oxygen before it reaches the sensor, which however involves even greater complexity.

Improving this state of the art is the main object of the invention, which is defined in the appended claims, where the dependent claims define advantageous variants.

Another object is to obtain an improved method and apparatus for detecting whether there is unwanted hydrogen in the oxygen stream generated as output by the PEM cell.

One aspect of the invention is a method for detecting the presence of (unwanted) hydrogen in the oxygen stream generated by a PEM cell, wherein the PEM cell comprises:

- a membrane permeable to $H^+$ ions,
- a first conduit for water entry,
- a second conduit for hydrogen exit,
- a third conduit for oxygen exit,

hydrogen and oxygen being produced by the molecular dissociation of water inside the PEM cell,
the method comprising the step of indirectly obtaining information regarding the presence of hydrogen in said oxygen stream by sensing the temperature of a catalyst, operating to facilitate and accelerate the recombination reaction between hydrogen and oxygen to form water, positioned in contact with said oxygen stream.

Another aspect of the invention relates to an apparatus comprising means for carrying out the method.

Another aspect of the invention relates to an apparatus, adapted to carry out the method, for detecting the presence of (unwanted) hydrogen in the oxygen stream generated by a PEM cell, wherein the PEM cell comprises:

- a membrane permeable to $H^+$ ions,

- a first conduit for water entry,
- a second conduit for hydrogen exit,
- a third conduit for oxygen exit,

hydrogen and oxygen being produced by the molecular dissociation of water inside the PEM cell, the apparatus comprising:

- a catalyst positioned in contact with the oxygen stream produced by the PEM cell,
- first means for sensing the catalyst temperature (e.g. a temperature sensor), and
- an electronic circuit configured to

  ▪ read a signal, generated by the first means, indicative of the catalyst temperature, and
  ▪ process such signal to indirectly obtain information relating to the presence of hydrogen in said oxygen stream.

*Catalyst* is generally meant here as a means or material that facilitates and accelerates the recombination reaction between hydrogen ($H_2$) and oxygen ($O_2$) to form water ($H_2O$), without being consumed in the process. The catalyst lowers the activation energy required for the reaction, allowing it to occur more rapidly and at lower temperatures than the uncatalyzed reaction.

Specifically, in the context of a PEM cell, the *catalyst* may be defined as a means or component that promotes the controlled recombination of hydrogen and oxygen within the gas stream exiting the PEM cell. The *catalyst* allows for indirect monitoring of the presence of hydrogen in the oxygen stream by detecting the heat generated by the catalyzed reaction.

The catalyst facilitates the recombination of oxygen and hydrogen in the third conduit, generating thermal energy as a result of this exothermal reaction. The catalyst temperature increases proportionally to the level of recombination, directly reflecting the amount of hydrogen present in said stream. This phenomenon allows, by measuring the catalyst temperature, not only to detect the presence of hydrogen in the stream but also to estimate its concentration: a higher catalyst temperature indicates a higher concentration of hydrogen in the stream.

The method and apparatus have significant advantages, for example:

1. Effectiveness in humid environments: the catalyst maintains its optimal functionality even in a humid environment or in the presence of liquid water, conditions typical of the oxygen output stream oxygen of the PEM cell. On the contrary, these conditions favor the sensor's operational efficiency;
2. Pressure Operation: The catalyst-based sensor can operate effectively even under high pressure conditions, a feature that significantly extends the range of applications and operating contexts in which the PEM device and/or cell can be used, increasing its versatility and usefulness in various industrial processes;
3. Practicality and affordability: the catalyst can be small in size and cost-effective, making it an efficient solution from both a space-saving and economic point of view;
4. Ease of implementation: the catalyst can be compact and versatile in nature, which allows it to be easily integrated into existing PEM cells, making it ideal for retrofitting operations without requiring substantial modifications to the system;
5. With a moderate content of hydrogen, the latter will recombine with oxygen to form water, thus reducing the risk of explosions in the volumes downstream of the sensor.

The above is especially true if one uses a single catalyst and a single temperature sensor associated with the catalyst.

To filter out the influence of temperature fluctuations of said stream from said information, thereby making the detection of the presence of oxygen more precise, the method preferably has the steps of also detecting the temperature of said stream and obtaining said information by determining or processing the difference between the detected temperature of the catalyst and the detected temperature of said stream.

Other advantages of the differential detection are the precise quantification of the hydrogen amount, because it provides a proportional measure of hydrogen presence; the compensation of room temperature; the reduction of false alarms due to environmental fluctuations; the real-time monitoring; and the predictive maintenance (intervention can be performed before the problem becomes critical).

With the same advantages, the apparatus preferably comprises second means for detecting the temperature of said stream.

In particular the electronic circuit is configured for

- reading a signal, generated by the first means, indicative of the catalyst temperature,

- reading a signal, generated by the second means, indicative of the temperature of said oxygen stream,
- obtaining indirectly information related to the presence of hydrogen in said oxygen stream from, or by processing, the difference between said two temperatures.

In one embodiment, the method comprises the step of heating the catalyst, preferably via a heating member, with the advantage of avoiding or reducing condensation that may form on it and improving the temperature reading.

In one embodiment the method comprises the steps of

heating the catalyst to raise its temperature by a known increment, and
obtaining said information by subtracting the known increment from the detected temperature of the catalyst, in particular obtaining said information

as a function of the difference between

the difference between the detected temperature of the catalyst and the detected temperature of said stream, and
said increment; and/or

as a function of the difference between
the detected temperature of said stream and
the sum of said increment and the detected temperature of the catalyst.

The advantage of said temperature increment is keeping the catalyst above the dew point, so it does not become humid. Another advantage is the greater sensitivity and stability of the measurement: the absence of condensation reduces thermal noise and makes the temperature increase due to $H_2$-$O_2$ recombination more readable. Furthermore, system calibration is easy because, knowing said temperature increment, the estimate of $H_2$ is obtained by compensation of the difference between the detected temperature of the catalyst and the detected temperature of the stream.

In particular, said temperature increment is constant or kept constant.

With the same advantages, the apparatus preferably comprises a heating member associated with the catalyst to raise the temperature of the catalyst.

In one embodiment the heating member is connected and/or powered to/by said electronic circuit.

In one embodiment the apparatus comprises an electronic circuit, preferably said electronic circuit, connected to the heating member to drive it in order to transfer a constant thermal power to the catalyst and/or absorb a constant thermal or supply power.

In one embodiment the apparatus comprises:

a heating member for raising the temperature of the catalyst by a known increment,
second means for detecting the temperature of said stream,
the electronic circuit being configured to indirectly obtain said information by subtracting
the known increment from the detected catalyst temperature.

In one embodiment the method comprises the steps of

raising the catalyst temperature by using a thermal power during operation of the PEM cell,
maintaining the catalyst temperature at a known, constant temperature value higher than the temperature of said stream by regulating the thermal power,
obtaining said information as a function of a variation of the thermal power.

The detection of the catalyst temperature is exploited to maintain such temperature at a constant value, in particular through a feedback loop.

In one embodiment the method comprises the steps of

heating the catalyst during operation of the PEM cell so as to maintain its temperature at a temperature value that is known and higher than the temperature of said stream,
measuring the thermal power supplied to the catalyst to maintain its temperature at said temperature value; and obtaining said information
as a function of the thermal power supplied to the catalyst, in particular as a function of a reduction of thermal power supplied to the catalyst which occurs in the presence of hydrogen with respect to a reference condition, more specifically as a function of a reduction of thermal power supplied to the catalyst which occurs in the presence of hydrogen with respect to a reference condition calculated as a function of the difference between the detected temperature of the catalyst and the detected temperature of said stream.

In one embodiment the method comprises the steps of

- heating the catalyst so as to maintain its temperature at a temperature value that is known and higher than the temperature of said stream,
- measuring the thermal power supplied to the catalyst in the absence of $H_2$ recombination in the catalyst;
- measuring the thermal power supplied to the catalyst in the presence of $H_2$ recombination in the catalyst; and
- obtaining said information as a function of the difference between said two thermal powers.

The advantage of this variant is to improve the repeatability and time-comparability of the $H_2$ measurement. Another advantage is the compensation for temperature variations in the stream present in the third conduit since measuring the thermal power supplied to the catalyst in the absence of $H_2$ recombination in the catalyst reduces the effect of fluctuations in the stream in the third conduit.

In one embodiment, said temperature value is kept constant.

In one embodiment, said temperature value is kept constant by a feedback control, e.g. a PID control, carried out by

detecting the temperature of the catalyst and
maintaining it at a reference value equal to said temperature value through the regulation of a thermal power emitted on or towards the catalyst and adapted to heat it.

With the same advantages the device comprises

means for measuring the thermal power supplied to the catalyst (e.g. a wattmeter) and
means for regulating the thermal power supplied to the catalyst so as to maintain its temperature at said temperature value; and
the electronic circuit is configured to determine said information
as a function of the thermal power supplied to the catalyst, in particular as a function of a reduction in thermal power supplied to the catalyst, more specifically
as a function of a reduction in thermal power supplied to the catalyst which occurs in the presence of hydrogen compared to a reference condition, more specifically as a function of a reduction in thermal power supplied to the catalyst which occurs in the presence of hydrogen with respect to a reference condition calculated as a function of the difference between the detected temperature of the catalyst and the detected temperature of said stream.

In one embodiment, the electronic circuit is configured to maintain said temperature value at a constant value.

In one embodiment, the electronic circuit is configured to maintain said temperature value through a feedback control, e.g. a PID control, by sensing the temperature of the catalyst and maintaining it at a reference value equal to said temperature value by regulating the thermal power emitted on or towards the catalyst and adapted to heat it, in particular the thermal power emitted on or towards the catalyst by the heating member.

In one embodiment the apparatus comprises:

- second means for detecting the temperature of said stream,
- a heating member for raising the temperature of the catalyst by using a thermal power;
- means for measuring said thermal power; and

the electronic circuit is configured for

maintaining the catalyst temperature at a constant value by regulating said thermal power;
detecting a thermal power value via said measuring means; and
obtaining indirectly said information as a function of the detected thermal power value and/or a variation of detected thermal power.

In one embodiment, the apparatus comprises means for providing a thermal power to the catalyst and/or to the heating member, and the electronic circuit is configured to regulate the means for providing a thermal power, preferably by feedback and/or by sensing the temperature of the catalyst, to maintain a constant temperature of the catalyst.

In one embodiment, the method comprises the step of installing the catalyst and the heating member in an insulated environment, thereby reducing heat losses from the heating member to the catalyst.

For the same purpose, in one embodiment the apparatus comprises an insulated environment in which the catalyst and the heating member are installed.

The step of heating the catalyst may be done in many ways.

In one embodiment, the method comprises the step of heating the catalyst by supplying it with a constant thermal power.

In one embodiment, the method comprises the step of heating the catalyst by supplying the heating member with a constant thermal power.

In one embodiment, the method comprises the step of heating the catalyst via an electrical resistor, an infrared emitter, or by coupling it to a heat exchanger, e.g. a fluid-operated heat exchanger.

In one embodiment the apparatus comprises an electrical resistor and/or an infrared emitter and/or a, e.g. fluid-operated, heat exchanger coupled to the catalyst to heat it.

Preferably the method comprises the step of

- evaluating the condition of presence of hydrogen in said stream as a function of the value of the detected catalyst temperature or of the value of said difference or of the value of said thermal power or of said thermal power reduction and/or of a value calculated from said information; and/or
- judging that the condition of presence of hydrogen in said stream is verified if the value of the detected catalyst temperature or of the value of said difference or of the value of said thermal power or of said reduction of thermal power and/or of a value calculated from said information, is greater than a threshold value; and/or
- assessing the presence of hydrogen in the third conduit on the basis of the value of the detected catalyst temperature or the value of said difference or the value of said thermal power or said reduction of thermal power and/or a value calculated from said information; and/or
- establishing the presence of hydrogen in said stream if the value of the detected catalyst temperature or the value of said difference or the value of said thermal power or said thermal power reduction and/or a value calculated from said information, is greater than a threshold value.

In particular, the step of evaluation of the presence of hydrogen on the basis of the detected temperature value of the catalyst or the value of said difference or the value of said thermal power or said reduction of thermal power and/or of a value calculated from said information with respect to a threshold is structured as follows:

- detecting the temperature of the catalyst in said stream or said difference or the value of said thermal power or said thermal power reduction and/or a value calculated from said information;
- comparing the detected temperature value or said difference or the value of said thermal power or said reduction of thermal power and/or a value calculated from said information, with a predetermined threshold value;
- if the threshold value is exceeded, the presence of hydrogen in said stream is deemed to have occurred.

Preferably, the method comprises the step of deciding whether a malfunction condition of the PEM cell is occurring as a function of the detected catalyst temperature value or said difference or the value of said thermal power or said reduction in thermal power and/or of a value calculated from said information; in particular, it comprises the step of establishing that the malfunction condition of the PEM cell is verified if the detected catalyst temperature value or said difference or the value of said thermal power or said reduction in thermal power and/or a value calculated from said information, is greater than a threshold value. That is, the method evaluates the condition that the PEM cell is malfunctioning as a function of the detected catalyst temperature value or said difference or the value of said thermal power or said reduction in thermal power and/or a value calculated from said information.

Specifically, the malfunction condition is a high amount of hydrogen in the oxygen stream generated by the PEM cell. More specifically, the malfunction condition is the perforation of one or more of the membranes in the PEM cell and/or the leakage of hydrogen produced by the PEM cell into the third conduit.

Preferably the method comprises the step of calculating the quantity of hydrogen in said stream, or in the third conduit, as a function of the detected temperature value of the catalyst or of said difference or of the value of said thermal power or of said reduction of thermal power and/or of a value calculated from said information, by processing the detected temperature value of the catalyst or of said difference or of the value of said thermal power or of said reduction of thermal power and/or of a value calculated from said information.

Preferably, if the temperature detected by the sensor associated to the catalyst or said difference or the value of said thermal power or said reduction of thermal power and/or a value calculated from said information, or the quantity of hydrogen in said stream or in the third conduit, reaches a threshold limit value, the method comprises the step of

generating a warning signal for a person and/or
deactivating the PEM cell.

In one variant, said threshold value is calculated by adding a temperature increment to the operating temperature value at which the oxygen exits the PEM cell under normal operating conditions. The increment may be a constant or proportional to the operating temperature value, e.g. a percentage thereof.

Said threshold value is preferably set to approximately 10 °C above the normal operating temperature (i.e. the temperature at which oxygen exits the PEM cell under defect-free conditions). In particular, PEM cells normally operate with a controlled water temperature, typically around 70 °C. Consequently, the oxygen exiting the PEM cell will have approximately this temperature. Taking this factor into account, for example with a normal operating temperature of 70 °C, a threshold value of 80°C (70°C + 10°C) is chosen. This setting allows:

taking into account normal temperature fluctuations of the system;
avoiding false alarms due to small variations in the operating temperature; and
detecting the presence of hydrogen when the recombination reaction causes a significant temperature increase.

The exact threshold value can, however, be calibrated based on the specific operating characteristics of each device, ensuring an optimal balance between detection sensitivity and robustness against false positives.

The catalyst used to recombine oxygen ($O_2$) and hydrogen ($H_2$) has the following preferred characteristics.

For the Shape :

- sphere or pellet shape, which offers a high specific surface area, favoring the efficiency of the catalytic reaction; or
- in the form of a coating on a support or conduit (such as metal monoliths or foams).

For the Composition:

- Metal catalyst, typically based on noble metals, in particular:

  a) Platinum (Pt): highly effective for $H_2$-$O_2$ recombination, with excellent corrosion resistance.
  b) Palladium (Pd): offers a good balance between catalytic effectiveness and cost, or
  c) other noble metals, which may include rhodium (Rh) or iridium (Ir), often used in combination with Pt or Pd.

For the Structure:

- The catalytically active metal is generally dispersed in the form of nanoparticles on a high-surface-area support (such as alumina, silica, or zeolites). This dispersion maximizes the catalyst's active surface area, increasing its efficiency.

The specific choice of catalyst (composition, shape, size) may be optimized based on the precise operating conditions of the apparatus and the PEM cell, and the sensitivity requirements in hydrogen detection.

Sometimes the water is pressurized in the first conduit, always at a lower pressure than the hydrogen. Preferably, the water is sent to the PEM cell through the first conduit, pushed, for example, by a pump.

In one variant, the catalyst is arranged inside the third conduit, for simplicity of construction and maximum interaction with the hydrogen stream.

Preferably, the apparatus comprises a gas/liquid separator downstream of the third conduit to separate the gases from the water (by gravity). Oxygen is released into the atmosphere as a waste product, while the water is, for example, recirculated in the PEM cell.

Specifically, the separator comprises a tank with two conduits: a bottom conduit for transporting water and an upper conduit for transporting moist oxygen. The catalyst and related first means are located inside the upper conduit.

Said catalyst and/or at least one of the sensors may be a tube or a conduit or the third conduit itself, through which the moist oxygen produced by the PEM cell passes; the catalyst does not necessarily have to be inside the tube itself or the third conduit.

Preferably the electronic circuit is a logic processing device or means, such as a PLC or a microcontroller.

Preferably, the electronic circuit is configured or programmed to perform all or some of the decision-making and/or operational steps of the method.

In particular, the apparatus comprises a generator connected to the PEM cell to power it electrically and controlled by the electronic circuit, the latter being configured so that when it detects a malfunction of the PEM cell through said means, it turns off the generator to deactivate the PEM cell.

In particular in the apparatus, the electronic circuit is connected to, or comprises, a signaling device capable of emitting a signal perceptible to a person (e.g. a display, a mobile phone, a PC, or a sound generator or an acoustic or luminous warning device).

Preferably the electronic circuit is configured to drive the signaling device to emit a warning signal when the temperature detected by the means associated with the catalyst, or said difference, exceeds said threshold value.

Preferably in the apparatus the electronic circuit is connected to, or comprises, a display device capable of displaying

the temperature value detected by the means associated with the catalyst or said difference or the value of said thermal power or said reduction of thermal power and/or a value calculated from said information; and/or

an indicative value of the estimated quantity of hydrogen within said stream, e.g. passing inside the third conduit or said upper conduit, and obtained by processing temperature information relating to the catalyst or to said difference or the value of said thermal power or of said thermal power reduction and/or of a value calculated from said information.

Preferably the apparatus comprises a flow controller, e.g. a valve, mounted on the first inlet conduit and controlled by the electronic circuit device, the electronic circuit being configured to drive the flow controller to inhibit operation of the PEM cell when it detects a malfunction of the PEM cell, particularly through the first and/or second means.

The catalyst is preferably a metallic catalyst, such as platinum- or palladium-based. Other preferred catalyst types include:

1. Bimetallic catalysts (e.g. platinum-ruthenium, palladium-gold);
2. Catalysts supported on metal oxides (e.g. platinum on titanium oxide);
3. Nanostructured catalysts (e.g. platinum nanoparticles); or
4. Zeolites containing noble metals.

The choice of catalyst may depend on factors such as recombination efficiency, thermal and chemical stability under operating conditions, and cost.

The connections between the electronic circuit and said means and/or the signaling device and/or the display device and/or the flow controller may be made via wired connections or wireless technologies such as Bluetooth Low Energy (BLE), Wi-Fi, ZigBee or other industrial wireless protocols.

The first and/or second means for detecting temperature preferably comprise a temperature sensor. One or each sensor is, for example, a resistive sensor (NTC, PTC), a thermocouple, an RTD or a semiconductor sensor, an infrared sensor, or a pyrometer.

The sensor of the first means is a sensor mounted in contact with the catalyst, or is detached from the catalyst, and is for example an infrared sensor or a pyrometer.

In general, any temperature sensing technology can be used for the sensor.

To detect said difference, it is not necessary to use two separate sensors to detect the two temperatures and then calculate the difference, a single sensor is sufficient to directly measure the difference, e.g. a thermocouple.

Another aspect of the invention relates to said apparatus, and a PEM cell associated with, and monitored by, said apparatus.

Preferably in the various aspects of the invention the adjustments and/or reading of the aforementioned physical variables are performed electronically.

The advantages of the invention will be made even clearer by the following description of preferred embodiments of the apparatus, which refers to the attached drawing in which:

- Fig. 1 shows a scheme of the apparatus;
- Figures 2-5 show variants for the scheme of the apparatus.

In the drawings, equal numbers indicate equal components and lines with arrows are fluid paths or signal lines.

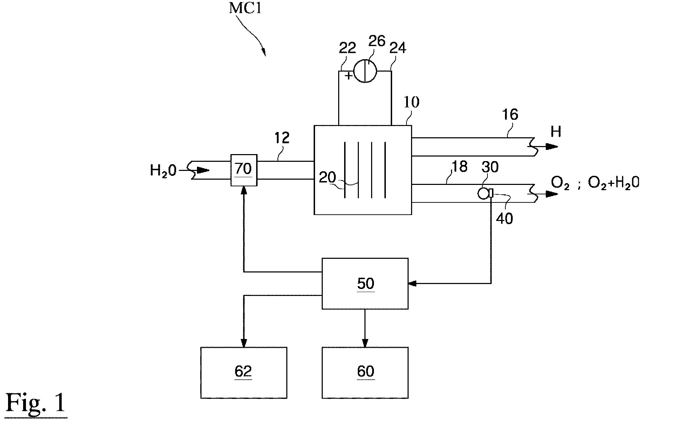

An apparatus MC1 (fig. 1) is designed to detect the presence of (unwanted) hydrogen in the oxygen stream generated by a PEM cell 10. The PEM cell 10 comprises an inlet conduit 12 for the water supply, an outlet conduit 16 for the transport of the generated hydrogen ($H_2$), and an outlet conduit 18 for the transport of the generated oxygen ($O_2$) (usually residual water is also present in the conduit 18, but not necessarily).

Inside the PEM cell 10 there are one or more known proton exchange membranes 20.

The PEM cell 10 is supplied with water, through the conduit 12, and electric current, supplied by an electric voltage generator 26 connected to terminals 22, 24 of the PEM cell.

The combined action of the generator 26 and the membrane 20 causes electrolytic dissociation of water

molecules, producing oxygen ($O_2$), which flows out of the PEM cell 10 through the conduit 18 together with residual water, and hydrogen ($H_2$), which flows into the conduit 16.

In the event of a rupture of a membrane 20 or a rupture of containment gaskets, contamination of the oxygen stream with hydrogen in the conduit 18 occurs.

To detect this anomaly, a catalyst 30 is arranged inside the conduit 18, with the function of recombining the hydrogen and oxygen present therein into water molecules. A sensor 40 detects the temperature $T_{cat}$ of the catalyst 30 and emits a signal indicative of such temperature, e.g. a voltage or current proportional to that temperature.

An electronic circuit 50 (e.g. a microcontroller or a PCB board) reads and processes the signal emitted by the sensor 40.

The operating logic is as follows: the greater the amount of hydrogen in the conduit 18, the greater the activity of the catalyst 30 and, consequently, its operating temperature $T_{cat}$. Therefore, there is a direct proportionality between the amount of oxygen in the conduit 18 and the temperature $T_{cat}$ of the catalyst 30. This relationship or proportion is used to determine the amount of hydrogen and/or whether the level of hydrogen inside the conduit 18 corresponds to an internal malfunction or damage of the PEM cell 10.

Preferably, the electronic circuit 50 reads the signal from the sensor 40 and evaluates, e.g. by comparing the signal with one or more thresholds, whether the malfunction condition is true or false.

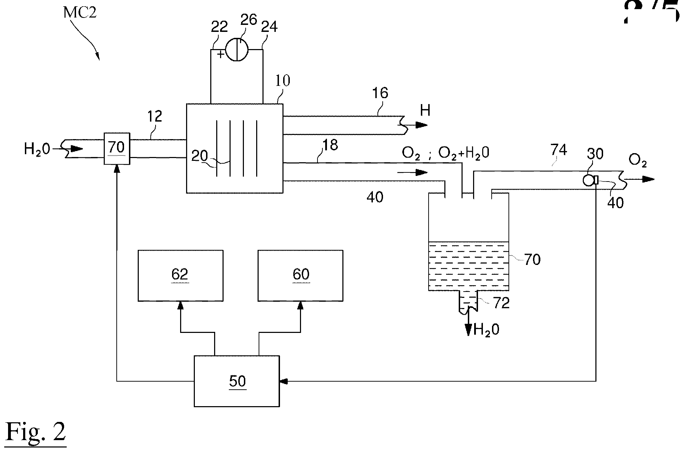

In a different variant (fig. 2), an apparatus MC2 downstream of the conduit 18 comprises a liquid/gas separator 70 that separates the water contained in the stream inside the conduit 18 from the gaseous components. For example, the separator 70 works by gravity, and is, for example, a tank into which the stream carried by the conduit 18 is injected and from which two conduits exit: a conduit 72 at the bottom that transports the water away and an upper conduit 74 that transports the moist oxygen away.

When the separator 70 is present in the system, the temperature readings are carried out in the upper conduit 74. The catalyst 30 and its sensor 40 are placed inside the conduit 74, with the advantage that the catalyst 30 operates in an environment that is at most humid but not submerged in water.

Preferably water is pumped from the conduit 72 into the conduit 12.

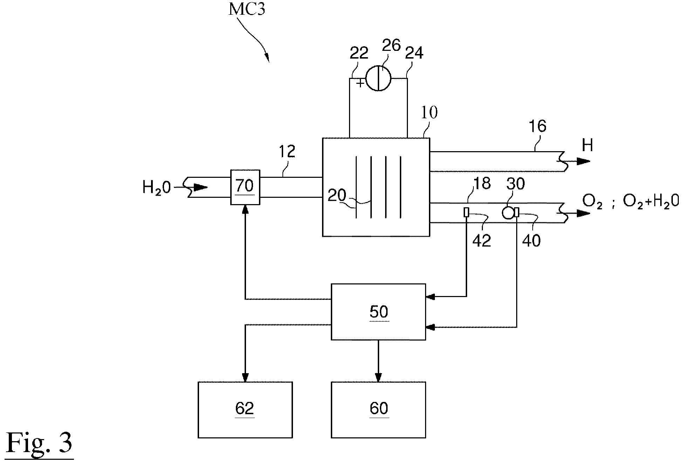

The apparatus MC1 and/or MC2 can be improved to measure/detect the amount of hydrogen in the oxygen stream generated by a PEM cell more precisely. For this purpose (fig. 3), a catalyst 30 and two temperature sensors 40, 42 are used in an apparatus MC3.

A first temperature sensor 40 is placed as shown in fig. 1 or 2 (adjacent or attached to the catalyst 30), a second temperature sensor 42 is placed upstream of the catalyst 30 with respect to the oxygen stream, e.g. in the third conduit 18 or in the conduit 74. The first temperature sensor 40 serves to detect the temperature $T_{cat}$ of the catalyst 30 while the second temperature sensor 42 serves to detect the temperature $T_F$ of the fluid flowing in the conduit 18 or 74. It is sufficient that the catalyst 30 does not significantly influence the second temperature sensor 42, therefore it is sufficient to distance them adequately. It is also possible to use a single temperature sensor to directly detect the temperature difference $\Delta T_{Ric}$ due to the recombination reaction

$$\Delta T_{Ric} = T_{cat} - T_F \text{ or } \Delta T_{Ric} = T_F - T_{cat}.$$

Based on the detected temperature difference $\Delta T_{Ric}$, the quantity of hydrogen present can be calculated/detected more precisely by establishing, even empirically or experimentally, a function between $\Delta T_{Ric}$ and the hydrogen concentration in the oxygen stream.

The variants of the apparatus MC2 may also be implemented in the apparatus MC3.

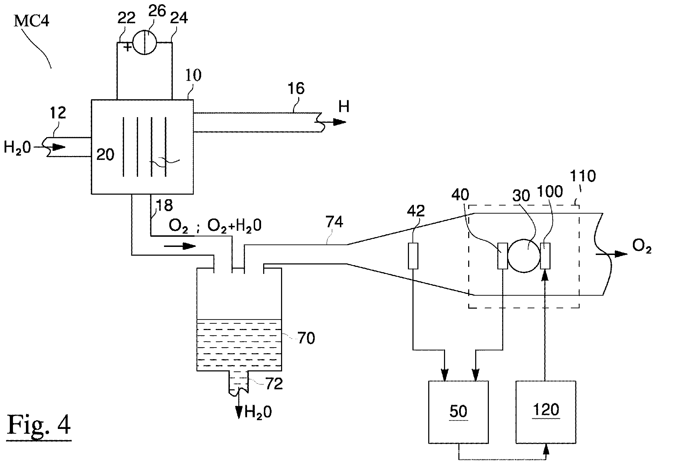

A variant for the apparatus MC1 or MC2 or MC3 is the apparatus MC4 in Fig. 4.

Even the apparatus MC4 preferably comprises a liquid/gas separator 70 downstream of the conduit 18 that separates the water contained in the stream within the conduit 18 from the gaseous components. For example, the separator 70 operates by gravity and is, for example, a tank into which the stream carried by the conduit 18 is injected and from which two conduits exit: a conduit 72 at the bottom that carries away the water and an upper conduit 74 that carries away the moist oxygen.

The apparatus MC4 comprises a heating member 100 associated with the catalyst 30 to heat the latter. The heating member 100 is adapted to raise the temperature $T_{cat}$ above the $T_F$ value by a known increment, with the advantage of preventing the formation of condensation and keeping the catalyst 30 dry.

For this purpose, the heating member 100 is connected to an energy supplier 120 which has the function of energizing the heating member 100 to make it emit heat towards the catalyst 30.

The temperature $T_{cat}$ is then

$$T_{cat} = T_F + \Delta T_H + \Delta T_{Ric},$$

where $\Delta T_H$ is the temperature increment in the catalyst 30 due to the action of the heating member 100.

To determine the amount of hydrogen, the following preferred algorithm is executed:

1. $T_{cat}$ and $T_F$ are measured;
2. $\Delta T_{Ric} = (T_{cat} - T_F) - \Delta T_H$ is calculated;
3. the concentration of $H_2$ is determined from $\Delta T_{Ric}$ with one of the methods described above, e.g. by using the relationship that the concentration of $H_2$ is proportional to $\Delta T_{Ric}$ and/or calculating a value as a function of said information.

The advantages of the apparatus MC4 are many.

The catalyst 30 always remains dry (without condensation) because by maintaining $T_{cat}$ at a $\Delta T_H$ above $T_F$ the catalyst 30 remains above the dew point and does not moisten. Furthermore, there is greater sensitivity and stability in the measurement of the $H_2$ concentration: the absence of condensation in the catalyst 30 reduces thermal noise and makes the temperature increment due to the $H_2$-$O_2$ recombination more readable.

The calibration of the system is simplified since by knowing $\Delta T_H$ the estimate of the $H_2$ concentration is obtained by simple compensation of $(T_{cat}-T_F)$.

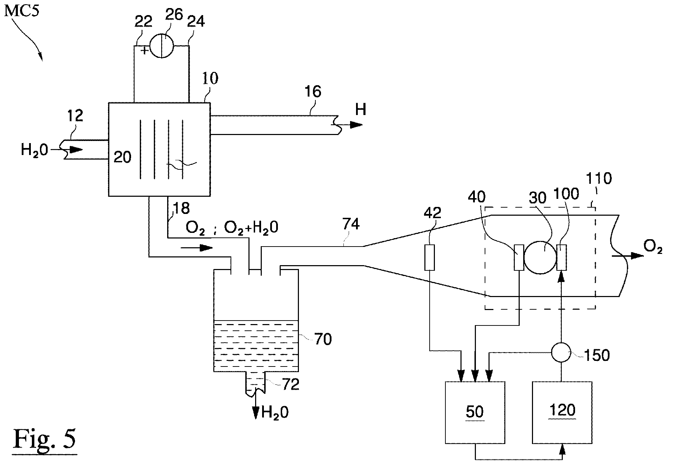

A variant for the MC1 or MC2 or MC3 or MC4 apparatus is the apparatus MC5 of fig. 5,

Even the apparatus MC5 preferably comprises a liquid/gas separator 70 downstream of conduit 18 that separates the water contained in the stream within the conduit 18 from the gaseous components. For example, the separator 70 operates by gravity and is, for example, a tank into which the stream carried by the conduit 18 is injected and from which two conduits exit: a conduit 72 at the bottom that carries away the water and an upper conduit 74 that carries away the moist oxygen.

The apparatus MC5 too comprises a heating member 100 associated with the catalyst 30 to heat it.

The apparatus MC5 also comprises

a power supplier 120 that is associated with the heating member 100 and that provides power to the heating member 100 for it to emit heat toward the catalyst 30; and
a power meter 150, connected to the circuit 50, for measuring the amount of thermal energy transferred per unit of time from the heating member 100 to the catalyst 30 or for measuring the amount of thermal energy transferred, or energy supplied, per unit of time to the heating member 100 (in the case of negligible heat losses toward the catalyst 30).

The heating member 100 operates to maintain the temperature $T_{cat}$ at a constant value and higher than $T_F$. For this purpose, the circuit 50 detects $T_{cat}$ with the sensor 40 and feedback controls the energy supplier 120 to maintain $T_{cat}$ at a predetermined constant value $T_{cat\_REF}$.

In particular, the circuit 50 performs a PID control to constantly maintain $T_{cat} = T_{cat\_REF}$.

When hydrogen is present in the conduit 74 (or 18 if there is no separator 70), the exothermal reaction $H_2+O_2\rightarrow H_2O$ generates heat, reducing the power required by the heating member 100 to maintain $T_{cat}$ at the value $T_{cat\_REF}$.

In the apparatus MC5 the thermal balance is:

$$P_H = K \cdot ( T_{cat\_REF} - T_F ) - P_{Ric}$$

where

K is a coefficient, to be determined even experimentally, which depends on the thermal losses of the system;
$P_H$ is the thermal power of the heating member 100;
$P_{Ric}$ is the thermal power of the exothermal reaction $H_2 +O_2 \rightarrow H_2O$.

To determine the amount of hydrogen in conduit 18, the following preferred algorithm is executed:

1. $P_H$ and $T_F$ are measured;
2. $P_{REF} = K\cdot( T_{cat\_REF} - T_F)$ is calculated;
3. $P_{Ric} = P_{REF} - P_H$ is determined;
4. the $H_2$ concentration is calculated as a function of $P_{Ric}$, e.g. with one of the methods described for the apparatus MC1 or MC2 or MC3 or MC4.

The advantages of this variant are many.

The temperature of the catalyst 30 is always constant, avoiding drifts. By maintaining $T_{cat}$ at a constant value the repeatability of the hydrogen measurement and the comparison of the various measurements over time is improved. The calculation method includes $T_F$ in the reference value $P_{REF}$, reducing the effect of stream fluctuations in the conduit 18.

It is understood that the options described only for one of the apparatuses MC1, MC2, MC3, MC4, MC5 can be implemented for each of the remaining ones, even where not specified.

In particular, the following advantageous options may be implemented in each of the apparatus MC1, MC2, MC3, MC4 and MC5.

Preferably, the sensor 40 and/or 42 is placed in contact with the catalyst 30, for simplicity of construction. For example, the sensor 40 and/or 42 is a resistive sensor (NTC, PTC), a thermocouple, an RTD, or a semiconductor sensor. Alternatively, the sensor 40 and/or 42 is detached from catalyst 30, and is, for example, an infrared sensor or a pyrometer. However, any temperature sensing technology can be used for the sensor 40 and/or 42.

Preferably, the sensor 40 and/or 42 is placed inside the conduit 18 or 74, but not necessarily.

Preferably, the apparatus MC1 and/or MC2 and/or MC3 comprises a flow controller 70, e.g. a valve, mounted on the inlet conduit 12 and controlled by the electronic circuit 50. Preferably, the flow controller 70 is configured to isolate the PEM cell 10 from the flow of water arriving from the inlet conduit 12. The electronic circuit 50 is configured so that when it detects a malfunction of the PEM cell 10, it drives the flow controller 70 to inhibit the operation of the PEM cell 10.

The catalyst 30 is preferably a metal catalyst, for example based on platinum or palladium.

The connections between the electronic circuit 50 and the sensor 40 and/or the sensor 42 and/or the signaling device 60 and/or the display device 62 and/or the flow controller 70 may be made via wired connections or wireless technologies such as Bluetooth Low Energy (BLE), Wi-Fi, or ZigBee. The choice of connection technology depends on factors such as the distance between the components, power consumption requirements, the need for data security, and the operating environment of the system.

Preferably, the electronic circuit 50 is connected to, or comprises, a signaling device 60 capable of emitting a signal perceptible to a person. For example, the signaling device 60 is a display, a mobile phone, a PC, or a sound generator or an acoustic or luminous warning device. Preferably, the electronic circuit 50 is configured to drive the signaling device 60 to emit a warning signal when the temperature detected by the sensor 40 or $\Delta T$ exceeds a threshold value. This allows a user to be warned that the cell 10 is malfunctioning.

Preferably the electronic circuit 50 is connected to, or comprises, a display device 62 capable of displaying

    the temperature value detected by sensor 40 or $\Delta T_{Ric}$ , and/or

    a value, indicative of the estimated quantity of hydrogen passing through the conduit 18 or 74, obtained by processing the temperature value detected by the sensor 40 or $\Delta T_{Ric}$.

This way, a user can be informed of how the cell 10 is performing.

Preferably, the generator 26 too is connected to and controlled by the electronic circuit 50. Then the electronic circuit 50 is configured so that when it detects a malfunction of the PEM cell 10, it turns off the generator 26 to deactivate the PEM cell 10.

Preferably water is pumped from the conduit 72 into the conduit 12.

The catalyst 30 and its sensor 40 are located inside the conduit 74, with the advantage that the catalyst 30 operates in an environment that is at most humid but not submerged in water.

As already mentioned, there is a direct proportionality between the quantity of oxygen in the conduit 18 or 74 and the temperature $T_{cat}$ of the catalyst 30. Furthermore, not only is there a direct proportionality between the quantity of oxygen in the conduit 18 or 74 and $\Delta T_{Ric}$, but this quantity, calculated or determined via $\Delta T_{Ric}$, is not affected by the temperature of the stream flowing in the conduit 18 or 74, nor by its fluctuations.

The electronic circuit 50 reads and processes the signal emitted by the sensors 40, 42. It then calculates $\Delta T_{Ric}$ in real time and converts $\Delta T_{Ric}$ into an estimate of the hydrogen concentration, e.g. via a *look-up table* or a calculation algorithm.

As a function of $\Delta T_{Ric}$, or of the result of the processing of $\Delta T_{Ric}$, the electronic circuit 50 can behave as already described for the apparatus MC1.

For example, the electronic circuit 50 may display the estimated hydrogen concentration on the display device 62, and/or may react to alarm thresholds based on the estimated concentration of hydrogen or on $\Delta T_{Ric}$.

The heating member 100 may have various embodiments, such as an electrical resistor, an infrared emitter, or a heat exchanger (e.g. fluid-operated).

The power supplier 120 is compatible with, or specialized for, the heating member 100. If the heating member 100 is an electrical resistor or an infrared emitter, the power supplier 120 is an electrical power-supply circuit. If the heating member 100 is a fluid-operated heat exchanger, the power supplier 120 is a heating means for the fluid.

The power meter 150 is compatible with, or specialized for, the heating member 100. If the heating member 100 is an electrical resistor, the meter 150 is an electrical wattmeter. If heating member 100 is a fluid-operated heat exchanger, the meter 150 is a calorie-counter.

Preferably, the catalyst 30 and the heating member 100 are contained in an insulated environment 110, to reduce heat losses. In this case, the sensor 40 may be installed inside the insulated environment 110 or on an outlet of the insulated environment 110 from which the hydrogen ($H_2$) introduced into the conduit 18 flows.

**Claims**

1. Method for detecting the presence of hydrogen in the oxygen stream generated by a PEM cell, wherein the PEM cell comprises:

   • a membrane permeable to $H^+$ ions ,
   • a first inlet conduit for water,
   • a second outlet conduit for hydrogen, and
   • a third outlet conduit for the generated oxygen,
   the hydrogen and the oxygen being produced by the molecular dissociation of water inside the PEM cell,
   with the step of indirectly obtaining information regarding the presence of hydrogen in said oxygen stream by sensing the temperature of a catalyst, operating to facilitate and accelerate the recombination reaction between hydrogen and oxygen to form water, placed in contact with said oxygen stream.

2. Method according to claim 1, with the steps of

   detecting the temperature of said stream and
   obtaining said information by determining the difference between the detected temperature of the catalyst and the detected temperature of said stream.

3. Method according to claim 1 or 2, wherein the catalyst is arranged inside the third conduit.

4. Method according to claim 1 or 2 or 3, with the further step of evaluating the condition of presence of hydrogen in said stream as a function of the value of the detected temperature of the catalyst and/or the value of said temperature difference.

5. Method according to claim 1 or 2 or 3 or 4, with the steps of

   heating the catalyst to raise its temperature by a known increment, and
   obtaining said information by determining the difference between the detected catalyst temperature and the known increment.

6. Method according to claim 1 or 2 or 3 or 4, with the steps of

   heating the catalyst to maintain its temperature at a constant value using a thermal power,
   measuring the thermal power,
   obtaining said information as a function of the thermal power or a reduction in the thermal power.

7. Method according to any preceding claim, with the additional step of judging as verified the presence of hydrogen in the oxygen stream produced by the PEM cell if the value of the detected catalyst temperature and/or the value of said temperature difference and/or a value calculated from said information is greater than a threshold value.

8. Method according to any preceding claim, comprising the step of judging that a PEM cell malfunction condition is occurring if the value of the detected catalyst temperature and/or the value of said temperature difference and/or a value calculated from said information is greater than a threshold value.

9. Method according to any preceding claim, with the step of generating a warning signal for a person and/or deactivating the PEM cell, if the detected temperature of the catalyst and/or said temperature difference and/or a value calculated from said information reaches or exceeds a threshold value.

10. Apparatus for monitoring the operation of a PEM cell, wherein the PEM cell comprises:

    • a membrane permeable to $H^+$ ions ,

• a first conduit for water entry,
• a second conduit for hydrogen exit,
• a third conduit for oxygen exit,
the hydrogen and oxygen being produced by the molecular dissociation of water inside the PEM cell,
the apparatus comprising:

• a catalyst, operating to facilitate and accelerate the recombination reaction between hydrogen and oxygen to form water, positioned in contact with said oxygen stream,
• first means for detecting the catalyst temperature, and
• an electronic circuit configured to

read a signal, generated by the first means, indicative of the catalyst temperature, and
process this signal to indirectly obtain information relating to the presence of hydrogen in said oxygen stream.

11. Apparatus according to claim 10, wherein the catalyst is positioned inside the third conduit.

12. Apparatus according to claim 10 or 11, comprising second means for sensing the temperature of said stream, the electronic circuit being configured to

• read a signal, generated by the first means, indicative of the catalyst temperature,
• read a signal, generated by the second means, indicative of the temperature of said oxygen stream, and
• indirectly obtain information regarding the presence of hydrogen in said oxygen stream from the difference between said two temperatures.

13. Apparatus according to claim 10 or 11 or 12, comprising

• a heating member for raising the temperature of the catalyst by a known increment, and
• second means for detecting the temperature of said stream,

the electronic circuit being configured to indirectly obtain said information by subtracting the known increment from the detected catalyst temperature.

14. Apparatus according to claim 10 or 11 or 12, comprising

• second means for detecting the temperature of said stream,
• a heating member for raising the temperature of the catalyst by using a thermal power;
• means for measuring such thermal power; and
the electronic circuit is configured for

maintaining the catalyst temperature at a constant value by regulating said thermal power;
detecting a thermal power value by said means for measuring; and
indirectly obtaining said information as a function of the detected thermal power value and/or of a detected thermal power variation.

MC1

$H_2O$

70

12

20

22 26 24

10

16 H

18 30

40

$O_2$ ; $O_2+H_2O$

50

62 60

Fig. 1

MC2

$H_2O$

22 26 24

10

12

16 H

18

$O_2$ ; $O_2+H_2O$

74 30 $O_2$

40

40

20

70

62 60

50

72

$H_2O$

**Fig. 2**

8 /5

MC3

22 26 24

10

12

16 H

$H_2O$

70

20

18 30

$O_2$ ; $O_2+H_2O$

42 40

50

62 60

## Fig. 3

**Fig. 4**

Fig. 5

Europäisches
Patentamt

European
Patent Office

Office européen
des brevets

# EUROPEAN SEARCH REPORT

**Application Number**

EP 25 19 9524

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | WO 2022/049089 A2 (ENAPTER S R L [IT])<br>10 March 2022 (2022-03-10)<br>* page 1, paragraph 3rd - paragraph 4th *<br>* page 9, paragraph 3rd *<br>* page 11, last paragraph *<br>* page 19, paragraph 4th *<br>* figures *<br>----- | 1,3-9,<br>13,14<br>2,12 | INV.<br>C25B1/04<br>C25B9/23<br>C25B15/027<br>C25B15/029<br>G01N33/00 |
| Y | GRIGORIEV S A ET AL: "Hydrogen safety aspects related to high-pressure polymer electrolyte membrane water electrolysis", INTERNATIONAL JOURNAL OF HYDROGEN ENERGY, ELSEVIER, AMSTERDAM, NL,<br>vol. 34, no. 14, 1 July 2009 (2009-07-01), pages 5986-5991, XP026351153,<br>ISSN: 0360-3199<br>[retrieved on 2009-02-14]<br>* page 5988 - page 5989 *<br>* figures 1,2 *<br>----- | 1-14 | |
| X | EP 1 279 640 A1 (DAVID SYSTEMS TECHNOLOGY SL [ES]) 29 January 2003 (2003-01-29) | 1-14 | TECHNICAL FIELDS SEARCHED (IPC) |
| Y | * paragraphs [0004], [0012], [0022], [0026] *<br>* paragraph [0038] - paragraph [0042] *<br>* claim 1 *<br>* figures 1,2 *<br>----- | 1-14 | C25B<br>G01N |
| X | WO 2017/154138 A1 (TOSHIBA KK [JP])<br>14 September 2017 (2017-09-14)<br>* paragraph [0042] - paragraph [0044]; figures 5,6 *<br>* paragraph [0052] - paragraph [0055]; figure 7 *<br>-----<br>-/-- | 1-14 | |

The present search report has been drawn up for all claims

| Place of search | Date of completion of the search | Examiner |
|---|---|---|
| Munich | 3 February 2026 | Ulivieri, Enrico |

Europäisches
Patentamt

European
Patent Office

Office européen
des brevets

**EUROPEAN SEARCH REPORT**

Application Number

EP 25 19 9524

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 6 036 827 A (ANDREWS CRAIG C [US] ET AL) 14 March 2000 (2000-03-14) * column 21, line 50 - line 55 * * column 24, line 17 - line 36 * * column 25, line 6 - line 60 * * figure 1 * | 1-14 | |
| A | SEIL KIM ET AL: "Thermochemical hydrogen sensor based on chalcogenide nanowire arrays", NANOTECHNOLOGY, IOP PUBLISHING, ENGLAND, vol. 26, no. 14, 19 March 2015 (2015-03-19), page 145503, XP020282888, ISSN: 0957-4484, DOI: 10.1088/0957-4484/26/14/145503 [retrieved on 2015-03-19] * Introduction; Conclusions * | 1-14 | |
| A | MIKHAYLOV A N ET AL: "Several Technical Solutions to Optimization of the Sensing Elements Design of the Thermocatalytic Hydrogen Sensors", 2020 WAVE ELECTRONICS AND ITS APPLICATION IN INFORMATION AND TELECOMMUNICATION SYSTEMS (WECONF), IEEE, 1 June 2020 (2020-06-01), pages 1-5, XP033787767, DOI: 10.1109/WECONF48837.2020.9131162 [retrieved on 2020-07-01] * equations, 2nd page; page 1st, column 1st; figures 1,2 * | 1-14 | TECHNICAL FIELDS SEARCHED (IPC) |

The present search report has been drawn up for all claims

| Place of search | Date of completion of the search | Examiner |
|---|---|---|
| Munich | 3 February 2026 | Ulivieri, Enrico |

EPO FORM 1503 03.82 (P04C01)

## ANNEX TO THE EUROPEAN SEARCH REPORT
## ON EUROPEAN PATENT APPLICATION NO.

EP 25 19 9524

03-02-2026

| Patent document cited in search report | | | Publication date | Patent family member(s) | | | Publication date |
|---|---|---|---|---|---|---|---|
| WO 2022049089 | A2 | | 10-03-2022 | AU | 2021338482 | A1 | 30-03-2023 |
| | | | | CA | 3190748 | A1 | 10-03-2022 |
| | | | | CN | 116322943 | A | 23-06-2023 |
| | | | | EP | 4208587 | A2 | 12-07-2023 |
| | | | | GB | 2598718 | A | 16-03-2022 |
| | | | | GB | 2605250 | A | 28-09-2022 |
| | | | | JP | 7769406 | B2 | 13-11-2025 |
| | | | | JP | 2023540050 | A | 21-09-2023 |
| | | | | KR | 20230095061 | A | 28-06-2023 |
| | | | | US | 2023264146 | A1 | 24-08-2023 |
| | | | | WO | 2022049089 | A2 | 10-03-2022 |
| EP 1279640 | A1 | | 29-01-2003 | AU | 3434000 | A | 08-10-2001 |
| | | | | BR | 0017205 | A | 08-04-2003 |
| | | | | CA | 2402966 | A1 | 04-10-2001 |
| | | | | CN | 1452591 | A | 29-10-2003 |
| | | | | EP | 1279640 | A1 | 29-01-2003 |
| | | | | JP | 2004500577 | A | 08-01-2004 |
| | | | | MX | PA02009701 | A | 27-03-2003 |
| | | | | US | 2003077202 | A1 | 24-04-2003 |
| | | | | WO | 0172633 | A1 | 04-10-2001 |
| WO 2017154138 | A1 | | 14-09-2017 | JP | 6574893 | B2 | 11-09-2019 |
| | | | | JP | WO2017154138 | A1 | 14-02-2019 |
| | | | | WO | 2017154138 | A1 | 14-09-2017 |
| US 6036827 | A | | 14-03-2000 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8669017 B